# EUROPEAN PATENT APPLICATION

(11) **EP 2 567 716 A1**
(43) Date of publication of application: **13.03.2013**
(21) Application number: 11775128.9
(22) Date of filing: 28.04.2011
(51) Int. Cl.: A61L 27/00

(54) **CELL INDUCTION MATERIAL**

(30) Priority: 28.04.2010 JP 2010104388
(71) Applicant: HI-LEX Corporation, Takarazuka-shi, Hyogo 665-0845 (JP)
(72) Inventor: YOSHINO, Kazutaka, Takarazuka-shi Hyogo 665-0845 (JP); AMETANI, Akihiro, Takarazuka-shi Hyogo 665-0845 (JP)
(74) Representative: Manitz, Finsterwald & Partner GbR
(86) International application number: PCT/JP2011/060408
(87) International publication number: WO 2011/136347

(57) **Abstract**

The object of the present invention is to provide a cell induction material, which can be easily formed, has excellent moldability because sintering is unnecessary, and decreases a strain on a human body caused by the exposure of the ends of a titanium wire. A metal mesh made by stockinette stitching with titanium wire is formed into a predetermined shape, and a cell induction material obtained by pressing the formed metal mesh is used. Further, the cell induction material with substantially no ends of the titanium wire that can be sensed through the sense in the vicinity of the surface is used.

## Description

### TECHNICAL FIELD

The present invention relates to a cell induction material which can be used for a material for medical use such as an artificial root, an artificial joint, an bone prosthesis and a skin terminal, especially to a cell induction material capable of inducing biological hard tissue or soft tissue as a scaffold.

### BACKGROUND ART

As prosthesis for replacing a defect site of human body with an artifact, metal material has been conventionally used for an artificial bone, an artificial root and the like. Since metal material has high strength, it is hard to resolve inside and outside a biological body and there is no possibility of deformation, etc., it is used for treatment with prostheses such as an artificial root, an artificial joint, an artificial bone and the like in the fields of surgery and dentistry. Because titanium and material made of titanium such as titanium alloy, among others, are less responsive to a foreign body in a biological body, relatively lightweight and non-magnetic, they are applied to a biological body broadly. While metal material has an advantage of having high strength, this strength makes it difficult to integrate with a biological body, and there was a difficulty in stabilizing metal material in a biological body, if used as it is. Therefore, for promoting the integration with a biological body and attempting stabilization in the biological body, metal porous body has been used for prostheses in order to induce biological tissue inside.

However, because metal porous body is difficult to manufacture by foaming, etc., not like resin porous body, and it is difficult to adjust the thickness of its cell wall, it can not be expected to be a material used for prostheses. Therefore, as a material superiorly stabilized in a biological body, scaffold material having a property inducing biological hard tissue is proposed. The scaffold material is formed into a laminated structure/into layers/is laminated by intertwining titanium or titanium base alloy fiber having a specific diameter and aspect ratio is suggested (Patent Document 1).

### PRIOR ART DOCUMENT

### Patent Document

Patent Document 1: JP 2004-067547 A

### SUMMERY OF THE INVENTION

### PROBLEM TO BE SOLVED BY THE INVENTION

However, the scaffold material has contact points and intersections between fibers by intertwining, in order to form apertures (cells) with such size to easily induce biological tissue. Therefore, sintering at a high temperature is required. Titanium, a material of the scaffold material, is likely to be brittle because it is inherently highly responsive to oxygen during heat treatment and easier to oxidize especially at a higher temperature. Accordingly, vacuum processing to a vacuum degree of 10⁻⁴ Pa or higher requiring a high level control and heat treatment under an inert gas atmosphere are necessary. Therefore, the processing must be performed with care and more sufficient process management including processing conditions, which complicates the steps for the processing. Moreover, because, for instance, titanium has a melting point of 1668°C and thus sintering requires equipment capable of very high temperature sintering in a high temperature environment, sintering work can not be performed easily and high energy cost is required. Moreover, when a brittle part of titanium fiber due to sintering comes off to expose ends of titanium wires constituting the titanium fiber or a plurality of ends of the titanium wires constituting the titanium fiber exist in the mesh of the titanium fiber, there is a concern about a strain on a patient such as inflammation caused by a stimulus to biological tissue when the end contacts the biological tissue.

In short, the object of the present invention is to provide the cell induction material which can form porous body easily, has superior formability because sintering is not necessary and reduces the strain on a human body due to the exposure of the ends of the titanium wires.

### MEANS FOR SOLVING THE PROBLEM

The present invention is directed to the cell induction material obtained by forming a metal mesh made by stockinette stitching with a titanium wire into a predetermined shape and pressing the formed metal mesh.

### EFFECTS OF THE INVENTION

By using the cell induction material of the present invention, the number of titanium wires constituting the cell induction material can be reduced, and moreover, there is no need for intertwining because stockinette stitch can form contact points and intersections between the titanium wire, as well. Therefore, because the cell induction material can reduce the number of the ends of the titanium wires in the cell induction material and does not cause coming off of the brittle portion due to intertwining to expose of the ends, no strain is put on a human body. Moreover, when the cell induction material of the present invention forms seamless tubular metal mesh, the cell induction material with less number of ends of a titanium wire can be provided by folding the metal tube. Moreover, when different wire other than the titanium wire constituting the metal mesh is used as a sewing material to obtain cell induction material by sewing the metal meshes formed into a predetermined shape onto each other or by sewing cell induction materials formed into a predetermined shape onto each other, even in the case of the deformation of the formed cell induction material by pressing, the porous body structure thereof does not collapse and the ends of the titanium wire are not exposed, and therefore the strain on a human body can be further reduced.

### BRIEF DESCRIPTION OF THE DRAWINGS

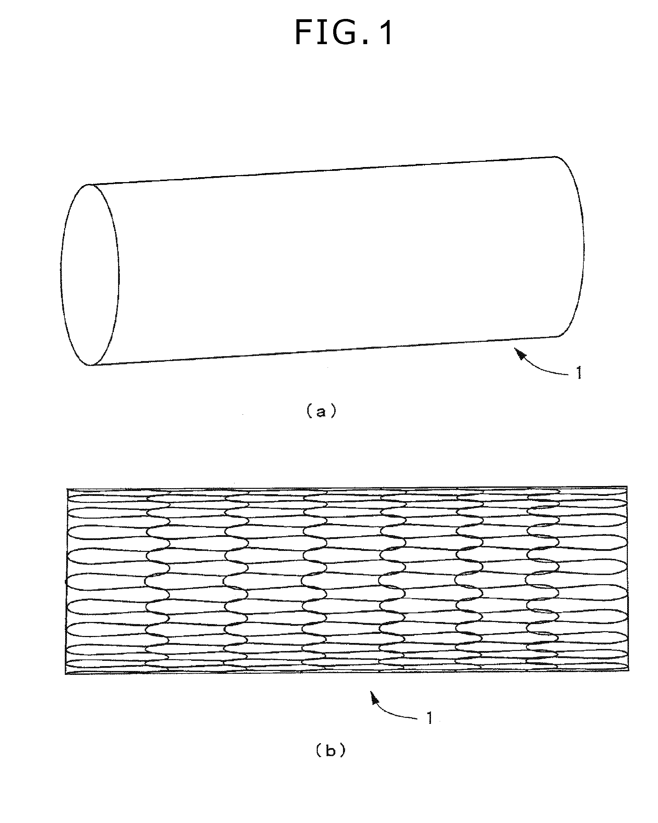

(FIG. 1) (a) An outline perspective view of the metal mesh constituting one embodiment of the cell induction material of the present invention. (b) A front view showing the metal mesh of Fig. 1 schematically.

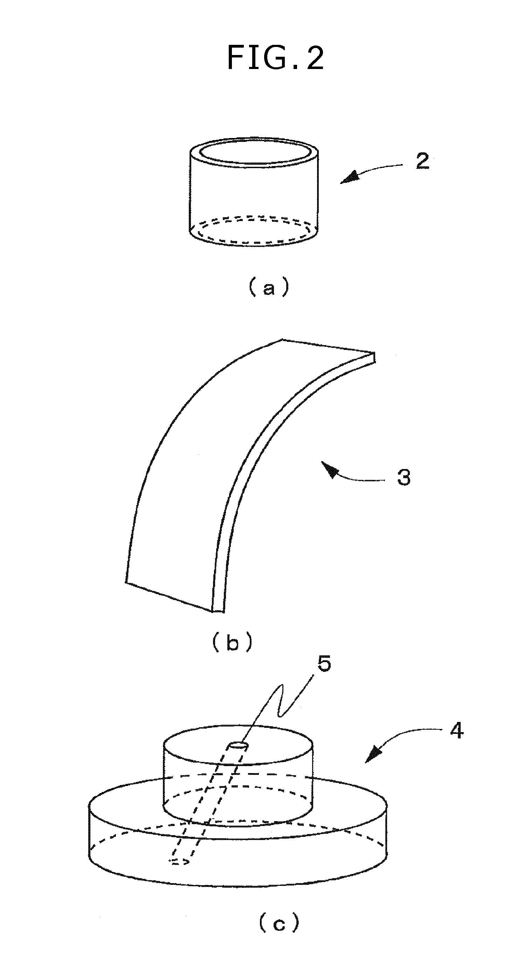

(FIG. 2) (a) A schematic view of the cell induction material of the present invention used for a blood vessel indwelling member, etc. such as a stent and a blood vessel substitute, etc. (b) A schematic view of the cell induction material of the present invention used for bone prosthetic material for a jawbone or a cranial bone. (c) A schematic view of the cell induction material of the present invention used for a skin terminal.

### EMBODIMENT FOR CARRYING OUT THE INVENTION

The cell induction material of the present invention will be described below with reference to drawings, but not limited thereto.

The present invention is a cell induction material obtained by forming a predetermined shape from metal mesh made by stockinette stitch with a titanium wire.
Regarding the cell induction material of the present invention, first, a metal mesh is formed by stockinette stitch with a titanium wire. Secondly, by pressing this metal mesh into a desired shape suitable for use, a scaffold having apertures (cells) with such size to easily induce biological hard tissue or soft tissue can be formed. Therefore, because the cell induction material of the present invention does not require intertwining, it is easy to form and because the cell induction material does not become brittle due to the deterioration caused by baking, there is no titanium wire end created by partly coming off.

The composition of the titanium wire used for the cell induction material 1 of the present invention is not especially limited as long as it is a wire that can be used for stockinette stitch such that the formed apertures (cells) have a size being able to induce cells as a scaffold, and it can be made of titanium metal or titanium alloy.

The metal mesh used for the cell induction material of the present invention is configured by stockinette stitch with titanium wire. Because the metal mesh has intersections of the wire formed by wire loops crossing and twining one another by being stockinette stitched, the intersections of the wire are flexible, and it has more stretchability, elasticity and flexibility compared to one intertwined or plainly woven. Therefore, there are more stretchability, elasticity and flexibility compared to the one formed by pressing a plainly woven metal mesh, even with the same hardness. Therefore, it can follow the motion of a human body when applied to the human body, and the cell induction material obtained by pressing can be used suitably for a prosthesis.

The metal mesh is not especially limited if formed by stockinette stitch with the titanium wire, and it is possible to suitably obtain cell induction material suitable for inducing cell tissue by using the metal mesh with a knitted textile fabrics width of 10 to 500 mm and with an aperture (knitted stitch size) of 2 to 6mm lengthwise and 1 to 5mm widthwise, for example. The diameter of titanium wire for obtaining such metal mesh is not especially limited, but a single wire with diameter of 10 µ m to 0.6mm, especially 50 to 150 µ m, or a wire configured by using a plurality of single wires such as a stranded wire configured by using single wires with diameter of 5 µ m can be suitably used to obtain the metal mesh with the above stitch size. The device, etc. used for knitting is not especially limited as long as stockinette stitch is employed as the knitting method, and the metal mesh configured by stockinette stitching with those titanium wires can be hand-knitted or a known device for stockinette stitching with metal wire can be used. In this regard, knitting stockinette stitch as described above is a method of knitting where a step of inserting a new loop through a ring-like loop of one or several titanium wires is repeated sequentially so as to knit a form of a fabric, and the method of knitting itself is not limited.

The metal mesh can be in a form suitable for use by pressing, and can be obtained by using a known press machine. Pressing condition to obtain the cell induction material from the stockinette stitched metal mesh is not especially limited as long as the cell size of the metal mesh enables cell induction and it is pressed so as to have hardness suitable for use. For example, by pressing with a condition of pressing pressure on the whole metal mesh set to 1 to 50t, especially 3t, so as to achieve the density of a compressed molding (bulk density) of 0.9 to 4.0g/cm³, the obtained cell induction material can be used suitably for a prosthesis. In this regard, the pressing can be performed on the metal mesh only, or on the metal mesh together with a metal molding body integrally, such as by winding onto a bar-shaped metal molding body, to integrate the cell induction material and the metal molding body with each other.

The metal mesh can be obtained as a seamless tube body by stockinette stitch. Stockinette stitch can provide a form of a planar fabric but stockinette stitch can also make the metal mesh a seamless tube body as shown in Figs. 1 (a) and (b). By having such tubular shape, the number of the ends can be reduced from four sides of a planar fabric to two sides, and thus a wire to prevent fraying from the ends of the metal mesh does not have to be used. Therefore, the metal mesh can reduce the number of ends of the titanium wire and is also superior in easiness of handling. A method to achieve a desired shape depending on the use by pressing the tube body is not especially limited, and it is also possible to place the tube body in a mold to press. However, it is also possible to obtain the cell induction material of the present invention by pressing with the aforementioned pressing condition after a specific shape is achieved by pressing the tube body with a lower pressure than the pressing pressure for obtaining the cell induction material in a desired shape, and then by folding the tube body. By the folding as described above, the pressing with the above pressing condition can be set easily, and it is possible to fold beforehand such that the ends of the titanium wire constituting the metal mesh come inside the molded body of the cell induction material. In the case of the folding beforehand in this way, by folding such that the ends come inside, the cell induction material pressed with the above pressing condition do not allow the titanium wire to come outside as an independent wire, with such wire substantially absent in the vicinity of the surface of the cell induction material. Therefore, the presence of the ends can not be sensed through the sense of touch even when human skin contacts the cell induction material. The cell induction material substantially having no ends of the titanium wire that can be sensed through the sense of touch in the vicinity of the surface to such a degree that the presence of the ends can not be sensed, when placed as a prosthesis in a human body, does not stimulate the biological tissue where it is placed, and there is no possibility of causing inflammation, etc. Therefore, the cell induction material can be used suitably as a cell induction material used as a prosthesis.

The cell induction material of the present invention can have a shape capable of fitting into the substrate of the stabilization part of a medical device that is stabilized in a biological body. With such shape, the substrate and the cell induction material do not have to be formed in integration with each other when obtaining the cell induction material, and physician and the like can combine the optimal substrate and the optimal cell induction material to match the shape, the size and the medical condition of an application part in accordance with physical features and medical condition of a patient. Therefore, because it is possible to combine the cell induction material in a medical setting, efficient treatment can be performed easily. The substrate is not especially limited as long as it is a substrate of the stabilization part in a biological body of a medical device having a function of being stabilized in a biological body such as by embedding. The medical device can be an implant part of an artificial root or a skin terminal, for example. The shape capable of fitting is not especially limited as long as the shape allows the cell induction material to fit into an object which the cell induction material is fitted into, and if the implant part is cylindrical, it can be shaped, for example as a tubular shape, being capable of fitting into the cylinder. In this regard, in order to fix the cell induction material to the substrate, engaging projections can be provided on the substrate so that engageable parts of the cell induction material such as apertures and ends are engaged with the engaging projections.

In addition, the cell induction material of the present invention can be a material where the metal mesh is fixed by pressing to the substrate of the stabilization part of the medical device stabilized in a biological body. The substrate is not especially limited as long as it is a substrate of a stabilization part of medical device having a function of being stabilized in a biological body. The substrate can be a part or the whole of the medical device, and is not limited in its shape or material. The cell induction material is not especially limited as long as the cell induction material is fixed to the substrate by pressing, and it is possible that the stockinette stitched metal mesh is formed beforehand and then pressed onto the substrate or the metal mesh is formed by stockinette stitching with titanium wire on the substrate and then pressed. The substrate is not especially limited with respect to material or shape as long as it allows the metal mesh to be fixed thereto as cell induction material by pressing, and thus the substrate can be fixed to the metal mesh by fusing together by pressing. The substrate can have engaging portions such as engaging projections or can be deformed due to pressing such that the metal mesh is engaging with and fixed to the substrate during pressing. Examples of the medical device with such substrate include a bone fixation material with a plate-like substrate having cell induction material with titanium wire molded densely on one surface by pressing, and having cell induction material with titanium wire molded sparsely on the other surface by pressing, the surfaces used separately for the stabilization for bone cells and the stabilization for cartilage cells, respectively.

As described above, the cell induction material of the present invention is made by pressing into a desired shape the metal mesh stockinette stitched from titanium wire, and the metal mesh can be made by stockinette stitching by one titanium wire. With one titanium wire constituting the metal mesh, the only ends of the titanium wire are the both ends of one titanium wire so that the presence of the ends can be minimized that can stimulate biological tissue when placed in a biological body. Therefore, the biological body is less stimulated and moreover, there is no need for the preparation such as folding the metal mesh so that the ends come inside the molded body of the cell induction material in order to reduce the stimulus to a biological body. Therefore, the cell induction material using the metal mesh made by stockinette stitching from one titanium wire can easily reduce the strain on a human body.

Moreover, the cell induction material of the present invention can use a wire, other than the titanium wire constituting the metal mesh, as a sewing material. As for the sewing material, titanium wire can be used as the material for maintaining the shape of the cell induction material molded by pressing the metal mesh, but material other than the titanium wire constituting the metal mesh can be used, and material other than the titanium wire constituting the metal mesh can be also used. The sewing material can be used in any way as long as it is used for maintaining the shape of the cell induction material molded by pressing the metal mesh, and the approximate contour of the cell induction material can be maintained while maintaining the stretchability, elasticity and flexibility of the cell induction material with the sewing material used to sew the cell induction material obtained by pressing. Moreover, a plurality of pressed metal meshes constituting the cell induction material can be sewn onto each other with the sewing material in order to prevent them from coming off. In this regard, a different wire other than the titanium wire constituting the metal mesh is a wire in a state of not constituting the metal mesh, and does not necessarily has to be separated from the wire constituting the metal mesh.

As the sewing material, although there is no limitation especially and a metal wire such as a titanium wire can be used, bioabsorbable suture such as ones made of polyglycolic acid and polylactic acid and non-absorbable suture such as ones made of nylon and polyester can be used. The materials for the sewing material can be used appropriately depending on use and purpose. For the cell induction material, such as a skin terminal, used with the assumption of the removal, easily available non-absorbable suture can be used, and for the cell induction material placed by being embedded into a biological body, the bioabsorbable suture can be suitably used because there is no need for suture removal as long as the purpose of the sewing material can be maintained. Regarding those sewing materials, known sewing materials such as the above sutures can be used, and sewing, including the use of a known sewing needle, is not especially limited as long as the metal meshes can be sewn onto each other or the cell induction materials can be sewn onto each other.

In addition, as materials other than the titanium wire constituting the metal mesh as described above, in the cell induction material of the present invention, a member allowing easy application to a therapeutic instrument used for treatment can be formed, including providing an attachment suitable for providing the cell induction material to a catheter if the cell induction material is a skin terminal, for example.

The use of the cell induction material of the present invention is not especially limited, if used as a prosthesis such as an artificial root, an artificial joint and an artificial bone in the fields of surgery and dentistry, and it can be used alone or in integration with other members. The cell induction material can be used, for example, as a bone prosthetic material, an artificial bone, an artificial joint, an artificial root and a connecting and holding member for ligament regeneration. The shapes for those uses are molded appropriately in accordance with the uses. A tubular cell induction material 2 as shown in Fig. 2(a) can be employed for a blood vessel indwelling member such as a stent and a blood vessel substitute, an indwelling portion in a human body in a region contacting blood such as an artificial esophagus, an artificial heart and an artificial trachea, and an artificial bone or a bone prosthesis material capable of containing bone marrow inside, for example. In the case of a bone prosthesis for a cranial bone or a jawbone, curved plate like cell induction material 3 as shown in Fig. 2(b) can be employed. In the case of a skin terminal, cell induction material 4 shaped to have a communication hole 5, through which a catheter, etc. can be inserted, as shown in Fig. 2 (c) can be employed. Moreover, the cell induction material of the present invention can be spherical, disc-shaped, cylindrical, rod-shaped or wedge-shaped. Those shapes can be formed by using a known method such as pressing and molding into shapes by using a metal mold and can be suitably used as a bone prosthesis material. The cell induction material of the present invention does not have to be intertwined by sintering it, thus is superior in stretchability, elasticity and flexibility compared with one intertwined, even when hardened by pressing, and can follow the motion of the human body. Therefore, it can be used suitably for medical devices applied to hard tissue such as a bone fixation material and a bone prosthetic material.

### Explanation of Symbols

1-4 Cell induction material
5 Communication hole

## Claims

1. A cell induction material obtained by forming a metal mesh made by stockinette stitching with titanium wire into a predetermined shape and then pressing the formed metal mesh.

2. The cell induction material according to claim 1, wherein the metal mesh is a seamless tubular body and the tubular body is folded to form the predetermined shape.

3. The cell induction material according to claim 1 or 2, wherein the metal mesh is made by stockinette stitching with one titanium wire.

4. The cell induction material according to any one of claims 1 to 3, wherein the cell induction material is not intertwined by sintering.

5. The cell induction material according to any one of claims 1 to 4, wherein the metal meshes formed into a predetermined shape are sewn onto each other or the cell induction materials formed into a predetermined shape are sewn onto each other, using a different wire other than the titanium wire constituting the metal mesh, as a sewing material.

6. The cell induction material according to any one of claims 1 to 5, wherein the cell induction material has substantially no ends of the titanium wire that can be sensed through the sense of touch in the vicinity of a surface.

7. The cell induction material according to any one of claims 1 to 6, wherein the cell induction material is shaped to be capable of fitting into a substrate of a stabilization part of a medical device that is stabilized in a biological body.

8. The cell induction material according to any one of claims 1 to 7, wherein the metal mesh is fixed by the pressing to a substrate of a stabilization part of a medical device to be stabilized in a biological body.
